# EUROPÄISCHE PATENTSCHRIFT

(11) **EP 1 313 755 B1**
(45) Veröffentlichungstag und Bekanntmachung des Hinweises auf die Patenterteilung: **21.11.2007**
(21) Anmeldenummer: 01980307.1
(22) Anmeldetag: 30.08.2001
(51) Int. Cl.: C07K 1/04, B01J 8/08

(54) **VERFAHREN UND FUNKTIONSPARTIKEL ZUR DURCHFÜHRUNG CHEMISCHER ODER BIOLOGISCHER REAKTIONEN ODER SYNTHESEN**
METHOD AND FUNCTIONAL PARTICLES FOR CARRYING OUT CHEMICAL OR BIOLOGICAL REACTIONS OR SYNTHESES
PROCEDE ET PARTICULES FONCTIONNELLES DESTINES A LA REALISATION DE REACTIONS OU DE SYNTHESES CHIMIQUES OU BIOLOGIQUES

(30) Priorität: 30.08.2000 DE 10043369
(43) Veröffentlichungstag der Anmeldung: 28.05.2003
(73) Patentinhaber: INSTITUT FÜR PHYSIKALISCHE HOCHTECHNOLOGIE E.V., 07745 Jena (DE)
(72) Erfinder: SCHOBER, Andreas, 86745 Schwangau (DE)
(74) Vertreter: Bock, Gerhard
(86) Internationale Anmeldenummer: PCT/EP2001/010033
(87) Internationale Veröffentlichungsnummer: WO 2002/018416

(56) Entgegenhaltungen:
- VENTON D L ET AL: "Screening combinatorial libraries" CHEMOMETRICS AND INTELLIGEMT LABORATORY SYSTEMS, ELSEVIER SCIENCE PUBLISHERS, AMSTERDAM, NL, Bd. 48, Nr. 2, 2. August 1999 (1999-08-02), Seiten 131-150, XP004171917 ISSN: 0169-7439
- FAUCHERE J-L ET AL: "Combinatorial chemistry for the generation of molecular diversity and the discovery of bioactive leads" CHEMOMETRICS AND INTELLIGEMT LABORATORY SYSTEMS, ELSEVIER SCIENCE PUBLISHERS, AMSTERDAM, NL, Bd. 43, Nr. 1-2, 28. September 1998 (1998-09-28), Seiten 43-68, XP004146894 ISSN: 0169-7439
- LAM K S ET AL: "The One-Bead-One-Compound Combinatorial Library Method" CHEMICAL REVIEWS, AMERICAN CHEMICAL SOCIETY. EASTON, US, Bd. 97, Nr. 2, März 1997 (1997-03), Seiten 411-448, XP002097485 ISSN: 0009-2665
- FENNIRI H ET AL: "TOWARDS THE DRED OF RESIN-SUPPORTED COMBINATORIAL LIBRARIES: A NON-INVASIVE METHODOLOGY BASED ON BEAD SELF-ENCODING AND MULTISPECTRAL IMAGING" ANGEWANDTE CHEMIE. INTERNATIONAL EDITION, VERLAG CHEMIE. WEINHEIM, DE, Bd. 39, Nr. 24, 15. Dezember 2000 (2000-12-15), Seiten 4483-4485, XP000975926 ISSN: 0570-0833

## Beschreibung

Die Erfindung betrifft ein Verfahren und Funktionspartikel zur Durchführung chemischer oder biologischer Reaktionen oder Synthesen, welche insbesondere im automatisierten Laborbetrieb im Bereich der kombinatorischen Chemie und molekularen Biotechnologie zum Einsatz gelangen.

Funktionskugeln, Beads sind als Support für chemische Synthesen eingeführt. Solche Partikel (beads) sind z.B. aus Glas oder Polystyrol ausgeführt. Festphasensynthesen an beads sind sowohl in der Oligonukleotidchemie und in der Peptidchemie als auch in der Synthese organischer Moleküle im makroskopischen Maßstab eingeführt. Hierbei wird der Festphasenträger in Säulen oder Platten verfüllt und entsprechend dem Syntheseprotokoll bzw. der eingesetzten Synthesevorrichtung mit Reagens gespült, benetzt gewaschen und die chemische Synthese induziert.

Auch in der biochemischen Forschung sind Festphasenträger (z.B. die sogenannten Dynabeads der Firma Dynal) als Träger biochemischen Materials eingeführt. Dabei wird das Trägermaterial z.B. mit Streptavidin beschichtet, was über eine Streptavidin Biotin Bindung ermöglicht, biotinylierte RNA oder DNA-Sequenzen an diese Träger zu binden. Dadurch ist es möglich, selektiv durch Waschen der Perlen mit dem gesuchten Target Bindungsexperimente durchzuführen.

Die chemische Synthese selbst wird an der Kugel, dem "bead" so ausgeführt, daß an einem sogenannten "Linker" (ein Kopplungsmolekül zur Festphase) die Ausgangsverbindung kovalent angebunden wird, welche entsprechend dem Syntheseprotokoll mit Reagenzien umgesetzt und bei Bedarf von dem Linker abgespalten werden kann. Die Kopplungsmoleküle können, je nach Ausführung, Phosphoamidite in der Oligonukleotidchemie oder die Aminosäuren in der Peptidchemie sein (Merryfield Synthese). Der Vorteil dieser Methode liegt in der Möglichkeit einen großen Überschuß an Reagens vorzulegen, sowie beliebig oft Kopplungs-, Wasch- und Entschützprozesse iterieren zu können.
Diese Vorteile führten auch zur Anwendung solcher Methoden in der organischen chemischen Synthese, bei der in gleicher Weise das zu koppelnde Molekül über einen Linker an die feste Phase synthetisiert wird.

Die Suche nach neuen Verbindungen mit gewünschten Funktionen wird vermehrt durch nicht-rationale Verfahren der Synthese von Leitstrukturen unterstützt, um zunächst eine große Anzahl diverser Strukturen zu synthetisieren. Dies betrifft die Synthese kleiner Moleküle durch chemische oder photosynthetische Verfahren, wie auch die Synthese größerer Moleküle wie Peptide, Proteine, Nukleinsäuren unter Ausnutzung chemischer oder enzymatischer Reaktionsverfahren. Durch die Verfügbarkeit miniaturisierter Analyseverfahren, durch die sich die sogenannte Fitness (Güte der chemischen oder biologischen Eigenschaften eines Syntheseproduktes, bezogen auf eine oder mehrere erwünschte Funktionen und Eigenschaften), feststellen bzw. quantitativ messen läßt, ist die Synthese dieser Leitstrukturen im Miniaturmaßstab möglich, wobei allerdings eine quantitative Produktanalytik zumeist erst hinterher durch Nachsynthese des Produkts in größeren Mengen erfolgen kann.

Begrenzender Faktor der oben beschriebenen, eingeführten Syntheseverfahren ist die zahlenmäßig limitierte Bereitstellung bzw. Synthese einer großen Anzahl diverser Moleküle.
Die chemische Synthese wird in den kommerziellen Synthesizern in Säulen oder planar über Pipettierroboter in Mikrotiterplatten an solchen Funktionskugeln durchgeführt. Der Parallelisierungsgrad überschreitet daher wesentlich nicht den Grad des bislang üblichen Microtiterstandards von 96 verschiedenen Proben. Die heutige Laborautomatisierung im Synthesebereich erreicht einen Parallelisierungsgrad von einigen hundert (z.B. Chem Speed 384) Proben im Volumenbereich von ca. 100 µl bis zu einigen ml (siehe z.B. DeWitt, A.W. Czarnik: Automated synthesis and Combinatorial chemistry Current Opinion in Biotechnology 1995, 6:640-645).

Die synthetisierten Substanzen liegen in makroskopischen Mengen pro Säule vor. Die maschinenbedingte Parallelität reicht jedoch nicht aus, um auch nur annähernd eine kombinatorische Vielfalt zu erreichen (z.B.: Oligonucleotidalphabet: 4ⁿ, Peptidalphabet: 20ⁿ, Organische libraries (α)ⁿ. Oligonukleotidstränge der Länge 6 summieren sich zu einer Vielfalt von rund 4000 Strängen, Oligopeptide der Länge 6 ergeben schon 64 Millionen verschiedene Substanzen).

Der im UHTS (UltraHighThroughput-Screening) eingeführte Ansatz bezieht sich auf die Durchführung automatisierter, miniaturisierter Screeningsysteme (einen Überblick bietet: Pauwels, H. Azijn, MP. de B, C. Claeys, K. Herzog "Automated techniques in biotechnology" Current Opinion in Biotechnology 1995, 6:111-117 oder Zhao, H., Arnold F.H. "Combinatorial protein design: Strategies for Screening Protein Libraries" 1997 Current Opinion in Structural Biology Vol. 7 S. 480-485, Burbaum, J.J; Sigal, N.M. "New Technologies for High-Throuput Screening" (1997) Current opinion in Chemical Biology, 1:72-78. Kommerziell ist diese Herangehensweise vor allem durch die Firmen Evotec, Hamburg und Aurora, San Diego bekannt geworden.
Der Bedarf an großen Substanzbibliotheken, die zum Testen den Screeningstrecken zugeführt werden, ist daher enorm gestiegen.

Ein anderer Weg große Molekülbibliotheken zu erzeugen, wird mit den sogenannten "Split and Pool" oder "Split and Mix"-Verfahren (siehe Lam et al. (1991) Nature 354:82, Glaser et al. (1992) J. Immunol., 149:3903-3913, Lam et al. (1993) Bioorg. & Med.Chem.Lett.3:419 und Sebestyen et al. (1993) Bioorg. & Med.Chem.Lett. 3:413) eingeschlagen.
Bei diesem Verfahren werden in jeweils n Synthesesäulen jeweils unterschiedliche Monomere gekoppelt. Danach werden die Mischungen in neuen Reaktionskammern "gepooled" d.h. vermischt, dann wieder definiert auf die jeweiligen Säulen verteilt und die unterschiedlichen Kopplungsschritte ausgeführt.

Der Vorteil dieses Verfahrens besteht in der Möglichkeit, ein breites Spektrum von verschiedenen Varianten zu synthetisieren, wobei jedoch verfahrenstechnisch sicherzustellen ist, daß auf jeder Perle exakt eine chemisch eindeutig zu charakterisierende Substanz erzeugt wird. Ein Nachteil besteht darin, daß zunächst nicht klar ist, welche Substanz sich auf welcher Perle befindet. Dies Problem wird allerdings im ersten Schritt von Bindungsexperimenten nicht akut. Erst selektierte Varianten müssen charakterisiert werden.

Um die Allgemeinheit des Verfahrens zu erhalten, wird daher daran gearbeitet die Charakterisierung indirekt durch Codierung oder direkt durch Analysenmethoden wie MS und MAS-NMR oder IR-Spektroskopie durchzuführen (W.L. Fitch, G. Detre, C.P. Holmes, J. Org. Chem.1994, 59, 7955. B.J. Egner, G.J. Langley, M. Badley, J.Org. Chem. 1995, 60, 2652).
Bei den meisten Verfahren der Codierung werden zusätzlich zu den Synthesebausteinen, entweder vor oder nach deren Kopplung, weitere, für jeden Synthesebaustein spezifische Verbindungen auf das Polymer gekoppelt. Diese als "tags" bezeichneten Markierungssubstanzen archivieren somit eindeutig den zeitlichen Syntheseablauf. Ein solches Kodierungssystem hat aber den entscheidenden Nachteil, kompatibel zu den eingesetzten Chemiesmus sein zu müssen, d.h. der Chemiker ist nicht frei in seinen Synthesewegen. Zudem erfordert die Analytik, d.h. die Dekodierung der chemischen tags, einen hohen logistischen Aufwand (S. Brenner, R.A. Lerner, Proc. Natl. Acad. Sci. 1992, 60, 5381. / Z.J. Ni, D. MacLean, C.P. Holmesm M.M. Murphy, B. Ruhland, J.W. Jacobs, E.M. Gordon, M.A. Gallop, J. Med. Chem. 1996, 39, 1601. / M.H.J. Ohlmeyer, R.N. Swanson, L.W. Dillard, J.C. Reader, G. Asouline, R. Kobayashi, M. Wigler, W.C. Still, Proc. Natl. Acad. Sci. 1993, 90, 10922. b) H.P. Nestler, P.A. Bartlett, W.C. Still, J. Org. Chem. 1994, 59, 4723).
Übliche kombinatorische "Split and Mix"-Verfahren sind also die schnellste Möglichkeit, das Problem der kombinatorischen Vielfalt zu lösen. Allerdings sind die Verfahren des "chemical Tagging" durch den erheblichen logistischen Aufwand im Anschluß an die Synthese stark eingeschränkt.

Um diese Probleme zu lösen, wird auf makroskopischer Ebene mit dem sogenannten "IRORI radio tag"-Verfahren, ein physikalischer Weg beschritten: Hierbei wird in einem Mikroreaktor, der mit Polystyrolkugeln befüllt ist, zusätzlich ein in Glas eingegossener, sogenannter "Radiofrequenztag", kurz RF-tag eingebracht. Der RF-tag besteht im wesentlichen aus einer Antenne und einem Transmitter bzw. Receiver. Jeder chemische Syntheseschritt und die entsprechende Einheit aus Reaktor und RF-tag sind eindeutig durch codierte Nummern charakterisiert. Die entsprechenden Nummern können sowohl geschrieben als auch gelesen werden. Damit ist der Prozeß vollständig beschrieben. Nachteile des RF-tag-Verfahrens sind sowohl verhältnismäßig hohe Kosten als auch die mangelnde Möglichkeit das Verfahren ohne großen Aufwand zu miniaturisieren.

Weiterhin sind miniaturisierte Verfahren bekannt. Bei der sogenannten AFFYMAX-Technik mit positionsspezifischen lichtinduzierten Kopplungsreaktionen (S.P.A. Fodor et al. (1991) Science 767-773) läßt sich eine spezifische Verbindung eindeutig aus ihrer jeweiligen x/y-Position bestimmen. Die Methode ist jedoch aus verschiedenen Gründen in ihrer Anwendungsbreite limitiert. Sie ist z.B. auf photoaktivierbare Kopplungsreaktionen beschränkt und erfordert umfangreiche Zwischen-und Waschschritte bei jeder Reaktion, da alle Reaktionspositionen jeweils mit allen eingesetzten Reagenzien in Kontakt kommen. Darüber hinaus muß die sequentielle Durchführung der Reaktionen bei Verwendung unterschiedlicher Reagenzien pro Reaktionsschritt als Nachteil angesehen werden.

In R. Frank, Tetrahedron Vol. 48, 42, 9217-9232, wird ein Verfahren beschrieben, nach dem es möglich ist, auf Cellulose-Filtern im Spot-Verfahren Bibliotheken von Festphasen-gekoppelten Peptiden in parallel geführten Synthesen zu erstellen, um sie anschließend einer funktionalen Prüfung zu unterziehen, z.B. der Bindung an einen Antikörper. Vergleichbare Verfahren wurden von Fodor et al. (Science 1991, 251, 767-773) und Geysen et al. (Proc. Natl. Acad. Sci, 1984, 81, 3998-4002) beschrieben. Andere Verfahren werden in einer Übersicht (Fields et al. Int. J. Peptide Protein Res. 1990, 35, 161-214) diskutiert. Die Kopplungs-Chemie ist, von Variationen abgesehen, für alle Verfahren im Prinzip vergleichbar und greift auf traditionelle Verfahren bzgl. Lösungsmittel, Schutzgruppenchemie und Aktivierung zurück. Vergleichbare Verfahren sind für die Oligonukleotid-Synthese entwickelt worden.
Die Ausbeuten sind dabei naturgemäß gering und einer makroskopischen Charakterisierung nicht mehr zugänglich.
Zusammenfassend ist zum bekannten Stand der Technik zu sagen, daß die parallele Synthese anhand der Koordinaten der betreffenden Rcaktionsgefäße (oder der Spots bei den miniaturisierten, flächigen Verfahren), das jeweilige entstandene Produkt identifiziert. Bei der kombinatorischen Synthese ist dagegen eine nachträgliche Bestimmung der Produktidentität erforderlich, außer es liegen, wie bei dem IRORI-Radio-tag-Verfahren, eindeutig bestimmbare physikalische Codierungen vor. Diesen bekannten Verfahren haften die vorstehend beschriebenen Nachteile an.

Der Erfindung liegt die Aufgabe zugrunde, ein Verfahren und dafür geeignete Funktionspartikel zur Durchführung chemischer oder biologischer Reaktionen oder Synthesen anzugeben, das eine vollständige Kompatibilität zwischen Mikro- und Makroscale gewährleistet, wobei eine große Vielfalt an Kopplungsreaktionen durchgeführt werden können und mit geringem Aufwand eine eindeutige Zuordnung der Reaktions-oder Syntheseprodukte zu den einzelnen Funktionspartikeln gewährleistet ist.

Die Aufgabe wird durch die kennzeichnenden Merkmale der Patentansprüche 1 und 4 gelöst.

Die Erfindung soll nachstehend anhand von Ausführungsbeispielen näher erläutert werden. Es zeigen:
- Fig.: 1 einen Teil eines verfahrensmäßigen Ablaufs für n Synthesereaktionsgefäße und n Funktionspartikel und

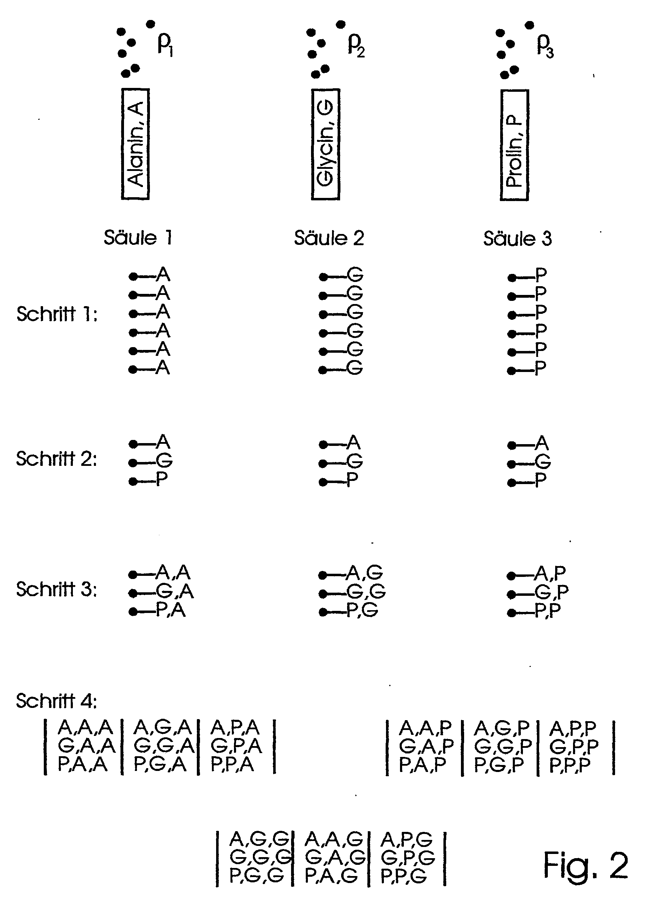

- Fig. 2: ein beispielhaften speziellen Verfahrensablauf zur Bildung einer Peptidbibliothek.

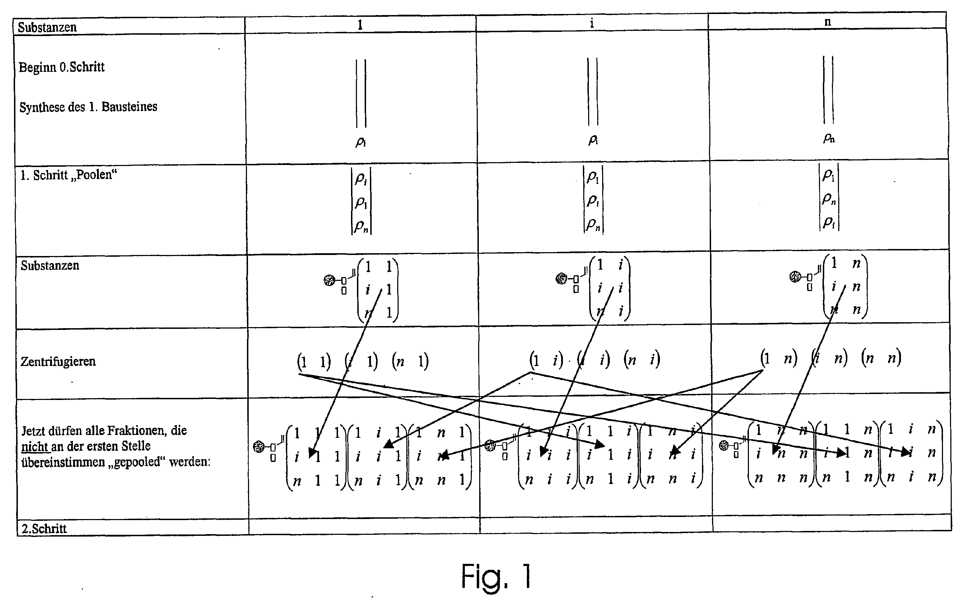

In einem ersten Ausführungsbeispiel nach Figur 1 sollen beispielhaft n Synthesereaktionsgefäße (z.B. Säulen) und n codierte Partikelchargen vorliegen. Die n Partikelchargen unterscheiden sich voneinander dadurch, daß sie eine voneinander abweichende Dichte ρ_{1...n} aufweisen, wobei die Dichte den Code darstellt. Damit lassen sich beliebige Substanzklassen ≤ n wie folgt synthetisieren:
Jedes der n Reaktionsgefäße soll im Beispiel jeweils mit unterschiedlichen chemischen Reaktionslösungen aus dem Pool der zu kombinierenden Bausteine beschickt (siehe Fig. 1) werden. Danach werden in jede Säule nach Möglichkeit die gleiche Anzahl von Funktionskugeln verbracht, wobei jeder Säule Funktionskugeln gleicher Dichte zugeordnet sind und die Dichten der Funktionskugeln, die den einzelnen Säulen zugeführt werden, voneinander abweichen.
Der erste Kopplungsschritt wird durchgeführt durch die Zuordnung von einer gegebenen Menge an Funktionspartikeln des Typs 1 zum Reaktionsgefäß 1, des Typs i zum Reaktionsgefäß i bis des Typs n zum Reaktionsgefäß n. Die Synthesekopplung (verschiedene Lösungsmittel, Protokoll etc.) wird vollzogen. Danach werden die Funktionskugeln den Reaktionsgefäßen entnommen, die jeweiligen Chargen entsprechend der Anzahl der weiter durchzuführenden Reaktionen, im Beispiel zu n gleichen Teilen, aufgeteilt und zu neuen Chargen derart zusammengestellt (gemixt), daß pro Charge eine Mischung aus Partikeln mit n unterschiedlichen Dichten vorliegt, d.h. bspw. in der i-ten Säule befinden sich anteilsmäßig Chargen aller n codierten Partikel und damit n verschiedene Substanzen. Im nächsten Syntheseschritt werden also in der Säule i alle Kombinationen aus (1.....i.....n) • i erzeugt. Diese Bibliothek kann nun jeweils in ihre Einzelkomponenten über ein Sedimentationsverfahren oder durch Zentrifugieren getrennt werden. Wichtig ist hierbei nur, daß sich die Zuordnung zum jeweiligen Schritt erhält bzw. der jeweiligen Säule erhält: also ((1,i); ...(i,i)...(n,i)) werden jeweils in Gefäße abgefüllt und die Numerierung bzw. Zuordnung gesondert registriert.
Im darauffolgenden Schritt werden nun alle Fraktionen, die nicht in der ersten Position übereinstimmen miteinander kombiniert, also ein Teil der Ausgangsfraktion wird mit i kombiniert ((1,i); ...(i, i);...(n, i))• i = ((1,i,i); ...(i, i, i);...(n, i, i)); ein Anteil der Fraktion z.B. der ersten Säule wird ebenso mit i kombiniert ((1, 1); (i, 1); (n, 1)) • i = ((1,1,i); (i, 1, i); (n, 1, i)) usw.
Mit diesem Verfahren kann man also alle möglichen Substanzkombinationen herstellen, wobei nur zwischen den einzelnen Schritten etwas Sorgfalt aufgewendet muß, um nicht Partikel gleicher Dichte und ähnlicher Historie zu kombinieren.
Vorstehend beschriebene Verfahrensweise der Trennung nach Partikeldichten nach erfolgtem Syntheseschritt und erneuter Chargenzusammenstellung ist solange durchzuführen, bis die gewünschten Reaktionskettenlängen erreicht sind, bzw. solange durchführbar bis pro zusammengestellter Charge jeweils nur noch ein Partikel pro vorgegebener unterschiedlicher Dichte vorliegt. Je nach gewünschter Synthese und der Anzahl vorgelegter Reaktionslösugen, ist ein Trennen der zusammengestellten Chargen nicht unbedingt nach jedem Syntheseschritt erforderlich, wie nachfolgendes Ausführungsbeispiel verdeutlicht.

In diesem zweiten Ausführungsbeispiel soll der einfache Fall einer Peptidbibliothek (wobei das vorgestellte Verfahren in keiner Weise auf peptidische oder sonstige Oligomere beschränkt ist), und der Einfachheit halber drei Fraktionen beschrieben werden:

Wie in Figur 2 dargestellt, sind drei Säulen vorgesehen, die jeweils mit Partikeln unterschiedlicher Dichte *ρ*1, *ρ*2, *ρ*3 beschickt werden. In der ersten Säule soll im Beispiel Alanin A, in der zweiten Säule Glycin G und in der dritten Säule Prolin P an die jeweiligen Polymerkügelchen gebunden werden. Nach Entnahme der Polymerkügelchen (Schritt 1) erfolgt eine Drittelung der jeweiligen Chargen und Zusammenmischung zu je drei weiteren Chargen, die jeweils Polymerkügelchen aller drei Ausgangschargen unterschiedlicher Dichte beinhalten (Schritt 2). Diese Chargen werden im Beispiel jeweils wieder den Säulen 1 bis 3 zugeführt, so daß in der ersten Säule die Synthesen A,A; G,A und P,A, in der zweiten Säule die Synthesen A,G; G,G und P,G und in der dritten Säule die Synthesen A,P; G,P und P,P erfolgen; das Verfahrensergebnis ist schematisch in Fig. 2 im Schritt 3 dargestellt. Im Beispiel sollen die so erhaltenen drei Chargen statistisch, d.h. ohne daß eine Auftrennung nach den unterschiedlichen Dichten der Polymerkügelchen erfolgt, in je drei Portionen aufgeteilt und jeweils eine Portion wiederum jeweils den Säulen 1 bis 3 zugeführt. Die so erhaltene Synthesevielfalt mit allen möglichen Substanzkombinationen ist in Schritt 4 von Fig. 2 dargestellt. Da diese Vielfalt der im Beispiel gewünschten Kettenlänge entspricht, erfolgte eine Auftrennung nach der Dichte der Polymerkügelchen erst nach dem letzten Syntheseschritt. Da die einzelnen Syntheseschritte protokolliert werden, ist eine exakte Zuordnung der Partikel und der daran synthetisierten Ketten auf einfache Weise möglich.

Die Trennung der synthetisierten Substanzen über die Dichtecodierung kann höchstgenau ausgeführt werden. Ultrazentrifugen sind z.B. noch in der Lage, zwei unterschiedliche DNA-Stränge, die sich nur an den natürlichen Positionen des ¹⁴N durch das Isotop ¹⁵N unterscheiden, aufzutrennen. Die Trennung komplementärer λ-Phagen DNA-Stränge ist ebenso möglich; 1.743 und 1.730 g/cm³. Für größere Partikel sind dagegen schon einfache Sedimentationsverfahren ausreichend. Je nach eingesetztem Trennungsverfahren und der Anzahl der durchzuführenden Syntheseschritte ist im Rahmen der Erfindung lediglich dafür Sorge zu tragen, daß die Dichteunterschiede der einzelnen Polymerkügelchen auch bei Beladung mit den Syntheseketten noch so groß sind, daß eine exakte Trennung nach Dichteunterschieden möglich ist. Die Beladung z.B. einer 100 µm Polystyrolkugel entspricht in etwa 100 pMol Synthesesubstanz. Nimmt man bspw. zwei Polystyrol/Komposit Kugeln mit einem Durchmesser von 100 µm und einer Dichte von 1 bzw. 1.1 g/cm³, das entspricht einem Gewicht von etwa 5.2 bzw. 5.72. 10⁻⁷g, und einer Beladung von 100 pMol mit einem mittleren Molekulargewicht von etwa 110, das entspricht einem Gewicht von etwa 10⁻⁸ g, ergibt sich in etwa eine Dichteänderung von 2%. Da sich die Dichten bei allen Kugelsorten im Mittel ändern und der Hauptbestandteil unverändert bleibt, ist das Verfahren breit anwendbar.

Der Hauptvorteil vorliegender Erfindung gegenüber den beschriebenen Lösungen nach dem Stand der Technik besteht darin, daß sich die Anzahl der erforderlichen Syntheseschritte auf die Anzahl der zum Einsatz gelangenden Dichtefraktionen reduziert und am Ende der Syntheseschritte eine exakte Zuordnung der Syntheseketten zu den einzelnen Funktionskügelchen gegeben ist.

Für die im vorgeschlagenen Verfahren zum Einsatz gelangenden Funktionskügelchen sind bspw. durch Suspensionspolymerisation gewonnene SiO₂-Partikel geeignet. Im Beispiel werden dabei 15 g SiO₂-Partikel mit einem Durchmesser im Bereich von 5-20 µm mit Methacrylolyloxypropyl-trimethoxysilan (0.5 ml, gelöst in 30 ml Toluol) 90 min unter Feuchtigkeitsausschluß bei 40°C umgesetzt. Danach werden die Partikel in einem Rotationsverdampfer im Vakuum bei Raumtemperatur getrocknet. In einem 11-Reaktor, versehen mit einem Rührer und einem Rückflußkühler werden 650 ml einer Lösung von 2 g Polyvinylpyrrolidon K90, 650 mg CaSO₄, und 100 mg Calciumphosphat vorgelegt. Bei 78°C werden 15 g vorbehandeltes Siliziumdioxid suspendiert in einem Gemisch aus 30 ml Styren, 0.6 ml Divinylbenzen und 400 mg Dibenzoylperoxyd zugegeben und bei einer Drehzahl von 500 U/min suspendiert. Nach Beendigung der Polymerisation nach 6 h werden die Kompositpartikel abgesaugt, gewaschen und durch Siebung klassifiziert. Auf diese Weise werden 43 g Partikel mit einem Durchmesser im Bereich von 100 µm bis 800 µm und einer Dichte im Bereich von 1.00 - 1.5 g/cm³ erhalten, aus denen nach Siebung und Dichtetrennung die für das Verfahren erforderlichen Chargen unterschiedlicher Dichte separierbar sind. Diese Partikel können, wenn erforderlich und wie nach dem Stand der Technik üblich, mit Ankergruppen für die temporäre Immobilisierung der ersten chemischen oder biologischen Komponente versehen werden.

Für bioanalytische Anwendungen sind auch Partikel einsetzbar, die wie folgt gewonnen werden: Eine Schmelze von Polystyrol in Toluol und eine Suspension von Siliziumdioxid (Partikeldurchmesser 5-10 µm) wird in einer Gradiendenmischkammer vermischt und sukzessive in eine gekühlte Vorlage mit Methanol vertropft. Auf diese Weise werden Partikel mit einem Durchmesser von 150 µm und einer Dichteveneilung im Bereich von 1g/cm³ bis 2.0 g/cm³ erhalten.

Diese Partikel sind löslich in einer Vielzahl organischer Lösungsmittel, jedoch unlöslich in Wasser. Beispielsweise können Proteine darauf immobilisiert und für Assays in wäßrigen Medien eingesetzt werden.

Ebenso liegt es im Rahmen der Erfindung, andere geeignete Funktionspartikel, auch solche unterschiedlicher Zusammensetzung oder Beschaffenheit, sofern sie inert gegen die zum Einsatz gelangenden Reaktionslösungen sind, einzusetzen, wenn die Maßgabe der unterschiedlichen Dichte der einzelnen Partikelchargen erfüllt ist.

## Patentansprüche

1. Verfahren zur Durchfiihrung chemischer oder biologischer Reaktionen oder Synthesen, bei denen eine Vielzahl von Funktionspartikeln, die eine Anbindung chemischer Komponenten oder Festphasensynthesen ermöglichen, zum Einsatz gelangen, **dadurch gekennzeichnet, daß** bei Einsatz von n Reaktionslösungen x Chargen von Funktionspartikeln vorgesehen werden, wobei x ≤ n ist, deren Dichte voneinander derart abweichend festgelegt werden, daß sie auch bei Beladung mit dem vollständigen Reaktions- oder Syntheseprodukt nach ihrer Dichte getrennt werden können, wobei
a) zunächst jede Charge einheitlicher Dichte einer ersten Reaktionslösung zugeführt wird, nach Anbindung des ersten Reaktionspartners
b) jede der x Chargen in maximal n Teilmengen aufgeteilt und eine Mischung der Teilmengen untereinander zu jeweils neuen Chargen, die Funktionspartikel aller Dichten beinhalten, hergestellt wird und
c) diese neuen Chargen jeweils einer weiteren Reaktionslösung zugeführt werden, wobei nach Durchführung der zweiten Reaktion bzw. Synthese
d1) entweder eine statistische Aufteilung der jeweiligen Teilchargen in jeweils wieder maximal n neue Untermengen erfolgt oder
d2) wenigstens einmal eine Trennung der erhaltenen Teilchargen nach ihrer Dichte vorgenommen wird und die jeweiligen Untermengen unterschiedlicher Herkunft wiederum miteinander zu neuen Teilchargen vermischt werden und
e) die Schritte nach c), d1) oder d2) solange wiederholt werden, bis die gewünschten Reaktions- oder Syntheseprodukte erhalten sind oder pro Untermenge jeweils nicht weniger als ein Funktionspartikel der n eingesetzten unterschiedlichen Dichten vorliegt, wobei
der Reaktions- oder Syntheseweg der Funktionspartikel in jedem der vorstehenden Verfahrensschritte protokolliert wird.

2. Verfahren nach Anspruch 1, **dadurch gekennzeichnet, daß** bei Durchführung mehrerer Reaktionen die erhaltenen Teilchargen von Funktionspartikeln unterschiedlicher Dichte mehrfach nach ihrer Dichte separiert und die so erhaltenen Untermengen zu neuen Teilchargen zusammengestellt werden.

3. Verfahren nach Anspruch 1, **dadurch gekennzeichnet, daß** bei Durchführung von x Reaktionen die erhaltenen Teilchargen von Funktionspartikeln unterschiedlicher Dichte nach jeder Reaktion nach ihrer Dichte separiert und die so erhaltenen Untermengen zu neuen Teilchargen zusammengestellt werden.

4. Funktionspartikel zur Durchführung chemischer oder biologischer Reaktionen oder Synthesen nach einem der vorstehenden Ansprüche, **dadurch gekennzeichnet, daß** in Abhängigkeit von der Anzahl n vorlegbarer Reaktionslösungen x Chargen jeweils einer größeren Anzahl von Funktionspartikeln vorgesehen sind, wobei x ≤ n ist, wobei die Dichte der einzelnen Chargen voneinander derart abweichend so groß festgelegt ist, daß die Funktionspartikel auch bei Beladung mit dem vollständigen Reaktions- oder Syntheseprodukt nach ihrer Dichte mittels geeigneter Separationsverfahren trennbar sind.

## Claims

1. Method for carrying out chemical or biological reactions or syntheses, whereby a great number of functional particles are used which enables a coupling of chemical components or solid phase syntheses, **characterized in that** when n reaction solutions are used then x charges of functional particles are provided, whereby x ≤ n, the density of which being determined differing from one other in such a way that they can be separated by their densities even when charged with the complete reaction or synthesis product, wherein
a) initially each charge of a homogeneous density is added to a first reaction solution, after bonding to a first reaction partner
b) each of said x charges is separated into maximally n partial sets and a mixture of the partial sets with one another to yield respective novel charges containing functional particles of all densities is produced, and
c) said novel charges each being added to a further reaction solution, whereby after completion of the second reaction and synthesis, respectively,
d1)either a statistical distribution of the respective partial charges again into respective maximally n novel subsets is carried out, or
d2)at least once a separation by density of the partial charges obtained is carried out and the respective subsets of different origin again are mixed with one another to yield novel partial charges, and
e) the steps c), d1) or d2) are repeated until the desired reaction products or synthesis products are obtained or, per subset, no less than one functional particle each of the n different densities used is obtained, whereby
the reaction or synthesis path of the functional particles in each of the above steps of the method is protocolled.

2. Method as claimed in claim 1, **characterized in that**, when carrying out a plurality of reactions, the obtained partial charges of functional particles of different densities are repeatedly separated by their density and the subsets obtained in this way are combined to novel partial charges.

3. Method as claimed in claim 1, **characterized in that** when carrying out x reactions, the obtained partial charges of functional particles of different densities are separated by their density after each reaction and the subsets obtained in this way are combined to novel partial charges.

4. Functional particles for carrying out chemical or biological reactions or syntheses according to one of the preceding claims, **characterized in that** in dependence on the number n of presentable reaction solutions x respective charges of a respective greater number of functional particles are provided, whereby x ≤ n, whereby the densities of the individual charges being determined such great differing from one other in such a way that, even when charged with the complete reaction or synthesis product, the functional particles can be separated by their density by way of suitable separation methods.

## Revendications

1. Le procédé destiné à la réalisation de réactions ou de synthèses chimiques ou biologiques avec participation d'une multitude de particules fonctionnelles permettant la liaison de composants chimiques ou de synthèses de phases solides, est **caractérisé en ce que**, dans le cas de l'utilisation de n solutions de réaction, on peut prévoir x charges de particules fonctionnelles, x étant égal à x ≤ n, dont la densité peut varier sensiblement d'une particule à l'autre afin qu'il soit possible de les séparer selon leur densité même en cas de chargement avec le produit complet de réaction ou de synthèse, en procédant de la manière suivante:
a) après l'entrée du premier partenaire de réaction, chacune des charges ayant la même densité est mise en contact avec une première solution de réaction;
b) chacune des x charges est subdivisée en au maximum n quantités partielles: se produit un mélange des quantités partielles entre elles pour former à leur tour de nouvelles charges composées de particules fonctionnelles présentant toutes les densités;
c) ces nouvelles charges sont chacune mises en contact avec une autre solution de réaction ; à la fin de la deuxième réaction ou synthèse
d1)se produit soit une subdivision statistique de chacune des charges partielles en, au maximum, n nouveaux volumes partiels, soit
d2)se produit au moins une fois une subdivision des charges partielles obtenues suivant leur densité et, ensuite, le mélange des différents volumes partiels d'origines diverses pour l'obtention de nouvelles charges partielles;
e) les opérations suivant c), d1): ou d2) sont répétées jusqu'à ce que les produits de réaction ou de synthèse requis soient obtenus ou bien jusqu'à ce que, pour tout volume partiel, il existe au moins une particule fonctionnelle présentant une des n différentes densités utilisées,
pour chacune des opérations du procédé décrites ci-dessus, le chemin de réaction ou de synthèse des particules fonctionnelles se trouve noté dans un compte rendu.

2. Le procédé suivant la revendication 1 est **caractérisé en ce que**, dans le cas de la réalisation de plusieurs réactions, les charges partielles obtenues de particules fonctionnelles de densité différente sont séparées à plusieurs reprises suivant leur densité pour former de nouvelles charges partielles à partir de ces nouvelles quantités partielles.

3. Le procédé suivant la revendication 1 est **caractérisé en ce que**, lors de la réalisation de x réactions, à la fin de chaque réaction partielle, les charges partielles des particules fonctionnelles de densité différentes sont séparées et que les quantités partielles ainsi obtenues forment de nouvelles charges partielles.

4. Les particules fonctionnelles destinées à la réalisation de réactions ou de synthèses chimiques ou biologiques suivant une des revendications précédentes sont **caractérisées en ce que**, en fonction du nombre n des solutions de réaction possibles, x charges d'un nombre important n de particules fonctionnelles en question sont prévues, x ≤ n , la densité de chacune des différentes charges étant fixée de manière à ce que les particules fonctionnelles puisse être séparées selon leur densité au moyen de procédés de séparation appropriés, même en cas de chargement avec le produit complet de réaction ou de synthèse.
